# EUROPÄISCHE PATENTSCHRIFT

(11) **EP 1 606 175 B1**
(45) Veröffentlichungstag und Bekanntmachung des Hinweises auf die Patenterteilung: **30.08.2006**
(21) Anmeldenummer: 04721795.5
(22) Anmeldetag: 19.03.2004
(51) Int. Cl.: B65D 5/74

(54) **FLACHER, NIEDRIGER AUSGIESSER-VERSCHLUSS MIT LAMINAT- ODER FOLIENWEGREISSER FÜR VERBUNDPACKUNGEN ODER MIT EINER DICHTFOLIE VERSCHLOSSENE BEHÄLTERÖFFNUNGEN**
LOCK FOR A FLAT AND LOW POURER PROVIDED WITH A REMOVABLE ELEMENT IN THE FORM OF A LAMINATE OF FILM FOR A COMPOSITE PACKAGE FOR CONTAINER OPENINGS CLOSED BY A NONPERMEABLE FILM
FERMETURE DE DISPOSITIF VERSEUR PLATE ET BASSE POURVUE D'UN ELEMENT ARRACHABLE SOUS FORME D'UN STRATIFIE OU D'UN FILM POUR EMBALLAGES COMPOSES OU POUR OUVERTURES DE CONTENANTS FERMEES PAR UN FILM ETANCHE

(30) Priorität: 26.03.2003 CH 52003
(43) Veröffentlichungstag der Anmeldung: 21.12.2005
(73) Patentinhaber: SIG Technology Ltd., 8212 Neuhausen am Rheinfall (CH)
(72) Erfinder: WASSUM, Markus, 78244 Gottmadingen (DE); WEIST, Mario, 79780 Stühlingen (DE)
(74) Vertreter: Felber, Josef
(86) Internationale Anmeldenummer: PCT/CH2004/000167
(87) Internationale Veröffentlichungsnummer: WO 2004/085272

(56) Entgegenhaltungen:
- WO-A-92/00884
- WO-A-03/080454
- DE-A- 4 409 945
- FR-A- 2 745 265
- US-A- 5 839 627

## Beschreibung

Diese Erfindung betrifft einen besonders flachen und niedrigen Ausgiesser-Verschluss für Verbundpackungen oder mit einer Dichtfolie verschlossene Behälteröffnungen, wobei er eine Einrichtung zum Wegreissen eines Stücks Verbundpackungs-Laminates oder eines Stücks Dichtfolie aus der mit dem Verschluss ausgerüsteten Verbundpackung oder aus der Dichtfolie der Behälteröffnung aufweist. Bei den mit diesem Verschluss auszurüstenden Verbundpackungen ist namentlich an Flüssigkeitspackungen in Form solcher Verbundpackungen aus folienbeschichtetem Papier gedacht, in denen etwa Milch, Fruchtsäfte, allerlei nichtalkoholische Getränke oder generell Flüssigkeiten auch aus dem Non-Food-Bereich verpackt werden. Der Verschluss kann aber auch für Verbundpackungen eingesetzt werden, in denen schüttfähige Güter wie etwa Zucker, Gries oder allerlei Chemikalien und ähnliches aufbewahrt bzw. verpackt werden. Bei diesem folienbeschichteten Papier handelt es sich um einen Laminatstoff, etwa um eine mit Kunststoff wie zum Beispiel Polyäthylen und/oder Aluminium beschichtete Papier- oder Kartonbahn. Gebräuchliche Volumina solcher Packungen reichen von 20cl bis zu 2 Litern und mehr. Zusätzlich kann dieser Ausgiesser-Verschluss aber auch auf Dichtfolien aufgeschweisst oder aufgeleimt werden, mit welchen irgend eine Behälteröffnung verschlossen ist, egal von welcher Art und Beschaffenheit der Behälter ist. Im Falle einer Verbundpackung wird an der Stelle, wo sich die lichte Öffnung des aufzuschweissenden Verschlusses befindet, aus der Verbundpackung ein zur Form der lichten Öffnung des Verschlusses passende Stelle vorbereitet, indem entweder der Lochrand eines entsprechenden Loches durch eine Stanzung geschwächt oder perforiert wird oder mittels einer Laserbehandlung die dortige Kartonschicht vorgeschwächt wird, wobei aber die Dichtfolie innerhalb des Verbundpackungs-Laminates unversehrt bleibt, oder aber ein passendes Loch komplett aus dem Laminat ausgestanzt wird, welches hernach einzig mit einer Dichtfolie wieder verschlossen wird.

Ausgiesser-Verschlüsse aus Kunststoff zum Aufschweissen auf eine derart vorbereitete Verbundpackung oder zum Aufbringen auf Dichtfolien sind in verschiedenen Ausführungen bekannt. Sie bilden einen Unterteil, welcher auf die Verbundpackung aufgeschweisst wird und einen daran über ein Schamier schwenkbar angeformten Deckelteil. Der Unterteil weist einen um die lichte Öffnung des Verschlusses nach oben auskragenden, rundumlaufenden Rand auf, der an der Vorderseite dieses Unterteils einen Ausgiess-Schnabel bildet. Der scharnierend an der Hinterseite des Unterteils angeformte Deckel weist eine nach unten ragende Ringwand auf, welche in zugeschwenktem Zustand des Deckels in den lichten Innenraum des Randes des Unterteils einpasst und innerhalb desselben einschappend einklickt, sodass der Ausgiesser-Verschluss dichtend verschliessbar ist. Beim erstmaligen Öffnen und somit beim erstmaligen Aufschwenken des Deckelteils kommt im Innern des nach oben auskragenden Randes des Unterteils die unterhalb des Unterteils verlaufende Stelle zum Vorschein, die entweder längs des Randes der lichten Öffnung des Verschlussunterteils vorgeschwächt ist, oder aber es kommt die Dichtfolie zum Vorschein, welche das dort herausgestanzte Loch in der Verbundpackung überspannt. Der Unterteil des Ausgiesser-Verschlusses ist ausserhalb des Loches mit seiner ebenen Unterseite auf die Verbundpackung aufgeschweisst. Standardgemäss wird die Verbundpackung also so gefertigt und vorbereitet, dass an jener Stelle, wo der lichte Innenraum des inneren Randes des Verschluss-Unterteils zu liegen kommt, in der Verbundpackung der Lochrand eines Loches vorgestanzt oder zumindest durch eine Perforierung geschwächt wird oder dort bloss eine Dichtfolie über das zuvor ausgestanzte Loch geklebt wird. Eine solche Dichtfolie besteht zum Beispiel aus einer Aluminiumfolie, welche auf der Innenseite der Verbundpackung mit der Kartonschicht der Packung verklebt ist. Sie kann aber auch von einer PE-Beschichtung gebildet sein, welche mittels einer Hochfrequenz-Schweissung auf das Kartonmaterial der Verbundpackung aufgeschweisst wird, sodass sie jeweils die Ausstanzung überspannt, auf welche der Ausgiesser-Verschluss später aufgeschweisst wird.

Wenn nun der Verschluss das erste Mal geöffnet wird, indem sein Deckelteil aufgeschwenkt wird, so kommt im Innern des nach oben auskragenden Randes des Verschluss-Unterteils je nach Ausführung eine randgeschwächte Stelle oder aber eine Dichtfolie zum Vorschein, sei es eine Alufolie oder eine PE-Folie. Die Folie oder randgeschwächte Stelle wird dann vom Benützer zunächst mit einem Finger eingedrückt, wonach der Inhalt der Verbundpackung durch das vom auskragenden Rand des Verschluss-Unterteils gebildete Ausguss-Stück über dessen an der Vorderseite ausgeformten Schnabel ausgiessbar ist.

Diese herkömmlichen Ausgiesser-Verschlüsse sind mit einigen Nachteilen behaftet. Zunächst mangelt den herkömmlichen Verschlüssen dieser Art und Gattung an, dass sie eine zu grosse Bauhöhe aufweisen, das heisst zu viel Höhe auf die damit ausgerüsteten Verbundpackungen auftragen, sodass die Möglichkeiten für die Stapelung solcher Verbundpackungen über Gebühr eingeschränkt werden und extra Abstandhalter vorgesehen werden müssen, um die Verbundpackungen in mehreren Lagen zu stapeln. Das aber macht einen Stapel um die Höhen der Abstandhalter bzw. mindestens um die addierten Höhen der Verschlüsse höher als theoretisch nötig wäre. Gerade die Stapelbarkeit in Regalen ist sowohl für die Grossisten wie auch für die Einzelhändler von grosser Bedeutung, denn Platz kostet Geld.

Weiter besteht ein wesentlicher Nachteil vieler herkömmlicher Lösungen darin, dass nach dem erstmaligen Aufschwenken des Deckelteils der Verschluss durch Eindrücken der randgeschwächten Stelle oder durch Eindrücken der Dichtfolie separat geöffnet werden muss und das mit dem Finger des Benützers geschieht. Diese Methode des Öffnens der Dichtfolie bzw. der Verbundpackung ist aber unhygienisch. Öffnet der Benützer die Verpackung mit unsauberen Fingern, so können Bakterien in den Verpackungsinhalt gelangen und sich darin vermehren und hernach werden sie, wenn es sich beim Inhalt um ein Lebensmittel oder ein Getränk handelt, von jemandem oral aufgenommen und können sich sodann unter Umständen infektiös auswirken.

Zudem wird von den herkömmlichen Verschlüssen die randgeschwächte Stelle oder die Dichtfolie regelmässig nicht sauber und vollständig aus dem lichten Bereich im Innern des auskragenden Randes herausgelöst. Vielmehr wird sie oftmals irgendwo in ihrem mittleren Bereich aufgerissen und dann werden die beiden verbleibenden Stücke oder Lappen links und rechts der Bruchlinie wenig gezielt nach unten in den Innenraum der Verbundpackung gedrückt. Es bilden sich dabei beidseits ausfransende Brauen, die nach unten in das Innere der Verbundpackung ragen und beim Ausgiessen den freien und sauberen Ausfluss des Inhaltes stören und beschränken. Wird die Verbundpackung allzu stark in Ausgiesslage geschwenkt, so vermag wegen der meist zu kleinen Dimensionierung der lichten Öffnung des Unterteils auch nicht hinreichend viel Luft in das Innere des Verbundpackung nachzuströmen. Das führt zu störendem Blubbern, das heisst zu einem unstetigen, schwallweisen Ausfliessen des flüssigen Inhaltes, was das gezielte dosierte Ausgiessen in ein Glas oder einen Becher erschwert. Desweiteren sind die Ausgiess-Schnäbel der Verschüsse oft nicht besonders vorteilhaft geformt, sodass beim Absetzen des Ausgiessens Flüssigkeit aufgrund von Kapillarwirkungen an der Aussenseite des Schnabels abwärts rinnt und dann über die Verbundpackung nach unten läuft. Dieses Kleckern des Ausgusses ist sehr ärgerlich, weil oftmals die ganze Vorderseite der Verbundpackung verunreinigt wird.

Der Deckelteil vieler herkömmlicher Verschlüsse wird zudem nicht zuverlässig von selbst in der Offenstellung des Deckels gehalten, sodass der Deckel infolge der Materialspannungen im Scharnierbereich langsam wieder zuschwenkt und den Ausgiess-Strahl stört, es sei denn, man hält den Deckelteil mit einer Hand bewusst in Offenstellung, was jedoch umständlich ist. In vielen Fällen wird die eine Hand zum Halten der Verbundpackung und zum Ausschenken benötigt, während die andere Hand etwa ein Glas hält, in welches eingeschenkt werden soll. Dann bleibt keine Hand zum Offenhalten des Deckelteils frei.

Viele herkömmliche Ausgiesser-Verschlüsse weisen ausserdem wenig bedienerfreundliche Garantieeinrichtungen auf, mittels derer die Erstöffnung, das heisst das erstmalige Aufschwenken des Verschluss-Oberteils, garantiert werden soll. Bei einigen Lösungen muss ein Garantieband weggerissen werden, welches mit zwei Fingern ergriffen werden muss. In der Praxis erweist sich das oft als schwierig. Wenn der Benützer zum Beispiel seine Hände kurz zuvor mit einer Hand- oder Sonnencrème behandelt hat, so gelingt es ihm schwerlich, das Garantieband wegzureissen, solange seine Finger fettig sind. Das Öffnen des Verschlusses mit Handschuhen ist erst recht nicht möglich. Schliesslich ist auch das Wiederverschliessen nicht befriedigend gelöst, weil die Verschlüsse nach dem Zuschwenken des Deckelteils nicht hinreichend dicht sind.
Aus der DE 44 09 945 A ist ein Ausgiesser-Verschluss bekannt, genannt Flachgiebelverschluss. Zum Öffnen dieses Verschlusses wird der Verschlussdeckel angehoben und um mehr als 90° aufgeschwenkt. Der Verschluss ist dann noch von einer Basisplatte verschlossen, die rundum von einer Einkerbung umschlossen wird. Oben auf der Basisplatte ist eine Handhabe angeformt. Zum Öffnen wird die Basisplatte durch Ziehen an der Handhabe weggerissen, unter Bruch des durch die Einkerbungen gebildeten schwachen Randes der Basisplatte, sodass ein vollständig freigelegtes Giessloch entsteht. Weil die Handhabe oben auf der Basisplatte angeformt ist, erhöht sich die Bauhöhe des Verschlusses entsprechend. Weiter zeigt die WO 03/080454 A einen ähnlichen Verschluss, bei welchem eine Lasche am Deckel angebracht ist, anstatt auf der Basisplatte. Der Deckel ist fest mit der Basisplatte verbunden und weshalb diese durch das Aufschwenken des Deckels mitgerissen wird.

Es gilt daher, diesen Problemen Abhilfe zu leisten und einen Ausgiesser-Verschluss für Verbundpackungen oder mit einer Dichtfolie verschlossene Behälteröffnungen zu schaffen, der bei geringster Bauhöhe ein hygienisches und für den Benützer leichteres und zuverlässigeres, sauberes Öffnen und vollständiges Entfernen der randgeschwächten Stelle oder der Dichtfolie erlaubt, welche die lichte Weite des Ausgusses überspannt, sodass hernach ein blubberfreies, kontinuierliches Ausgiessen mit einem dickeren Flüssigkeitsstrahl ermöglicht wird. Ausserdem soll der Verschluss in Offenstellung verbleiben, indem sein Deckel nicht von selbst auf den Unterteil zurückschwenken kann und schliesslich soll der Verschluss durch Niederschwenken des Deckels auf den Unterteil hinreichend verschlossen werden können, sodass nicht leicht Flüssigkeit aus der Verbundpackung ausrinnen kann.

Die Aufgabe wird gelöst von einem flachen und niedrigen Ausgiesser-Verschluss für Verbundpackungen oder mit einer Dichtfolie verschlossene Behälteröffnungen, bestehend aus einem Unterteil, welcher mit der Unterseite seiner Grundplatte auf eine Verbundpackung oder Dichtfolie aufzuschweissen oder aufzuleimen bestimmt ist, wobei die Grundplatte eine Durchflussöffnung aufweist sowie eine diese Grundplatte aussen umlaufende, nach oben ragende Auskragung, sowie aus einem zu diesem Unterteil über ein Scharnier schwenkbar angeformten Deckel zum Aufschwenken und dichtenden Niederschwenken auf den Unterteil, mit in die Auskragung am Unterteil einpassendem rundumlaufenden Kragen auf der Unterseite des Deckels, wobei an der Grundplatte in deren lichter Durchflussöffnung eine mit ihrem Aussenrand dem Innenrand der Durchflussöff nung nachgeführte Wegreissplatte über Materialbrücken als Sollbruchstellen angeformt ist, sodass die Unterseite der Wegreissplatte mit der Unterseite der Grundplatte bündig verläuft, wobei er sich dadurch auszeichnet, dass die Wegreissplatte eine Aussparung aufweist, in welcher an einer Randstelle eine Lasche angeformt ist, an welcher die Wegreissplatte nach Aufschwenken des Deckels unter Bruch der Materialbrücken aus der Durchflussöffnung herausreissbar ist.

In den Figuren ist eine vorteilhafte Ausführung dieses besonders flachen und niedrigen Ausgiesser-Verschlusses für Verbundpackungen oder für mit Dichtfolie verschlossene Behälteröffnungen in verschiedenen Ansichten dargestellt. Anhand dieser Figuren wird dieser Ausgiesser-Verschluss nachfolgend im einzelnen beschrieben und seine Funktion wird erläutert und erklärt.
Es zeigt:
- Figur 1:: Den flachen und niedrigen Ausgiesser-Verschluss in geschlossenem Zustand von schräg oben in perspektivischer Ansicht gesehen;
- Figur 2:: Den Ausgiesser-Verschluss nach Figur 1 in geschlossenem Zustand, jedoch in einem Längsschnitt von schräg oben in perspektivischer Ansicht gesehen;
- Figur 3:: Den Ausgiesser-Verschluss nach Figur 1 mit um 180° aufgeschwenktem Deckel in noch ungeöffnetem Zustand der Verbundpackung, das heisst mit intakter Wegreissplatte, in perspektivischer Ansicht von schräg oben gesehen;
- Figur 4:: Den Ausgiesser-Verschluss nach Figur 1 mit um 180° aufgeschwenktem Deckel in noch ungeöffnetem Zustand der Verbundpackung, das heisst mit intakter Wegreissplatte, in perspektivischer Ansicht von unten gesehen;
- Figur 5:: Den Ausgiesser-Verschluss nach Figur 1 mit um etwa 60° aufgeschwenktem Deckel und in geöffnetem Zustand der Verbundpackung, das heisst mit weggerissener Wegreissplatte, in perspektivischer Ansicht von schräg oben gesehen.

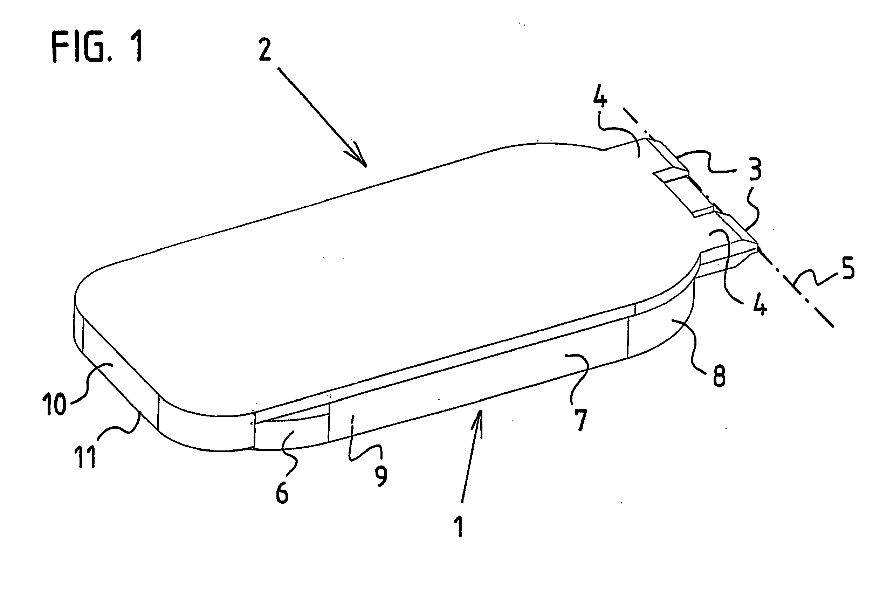

In Figur 1 ist dieser besonders flache und niedrige Ausgiesserverschluss aus gespritztem Kunststoff für eine Verbundpackung in einer Gesamtansicht in geschlossenem Zustand in Perspektive gezeigt. Der gleiche Verschluss kann jedoch auch auf eine Dichtfolie aufgeschweisst oder aufgeleimt werden, mit welcher die Behälteröffnung irgendeines Behälters verschlossen ist. Um eine Grössenordnung dieses hier doch in einer wesentlich vergrösserten Abbildung dargestellten Verschlusses anzugeben, seien folgende Masse genannt: Die Breite beträgt zum Beispiel ca. 20mm, die Länge ca. 45mm und die Höhe über alles bloss ca. 4mm, und das alles bei einer Materialstärke für Deckel und Unterteil von bloss ca. 1 mm. Es versteht sich gleich zu Beginn, dass diese Masse nicht verbindlich sind und es klar ist, dass auch noch kleinere Verschlüsse oder auch grössere auf der Basis der hier offenbarten technischen Lehre realisierbar sind, mit abweichenden Massbeziehungen von Länge, Breite und Höhe zueinander. Man erkennt am hier gezeigten Verschluss bloss den Unterteil 1 mit dem darauf geschwenkten Deckel 2 sowie die für die Schwenkung nötigen Filmscharniere 3, welche gegenüber den Verschlussteilen Unterteil 1 und Deckel 2 über die Scharnierhebel 4 etwas zurückversetzt angeordnet sind. Die Scharnierachse 5 ist deswegen gegenüber den zu verschwenkenden Teilen zurückversetzt, damit beim Zuschwenken des Deckels 2 auf den Unterteil 1 auch im hinteren Bereich des Deckels 2 von diesem ein Weg zurückgelegt wird, welcher es ermöglicht, Schnappeinrichtungen vorzusehen, sodass die beiden Teile ineinander einklicken können und somit ein dichter Verschluss des Deckels 2 auf dem Unterteil 1 gewährleistet werden kann. Der Unterteil 1 des Verschlusses weist eine nach oben ragende Auskragung 6-8 auf, welche seine Grundplatte 9 rundum einfassen. Der Unterteil 1 bildet im Grundriss die Form einer Null, also im wesentlichen eine Rechteckform mit abgerundeten Ecken. Entsprechend schliesst die am Aussenrand nach oben ragende Auskragung gerade Abschnitte 7 auf wie auch gekrümmte Abschnitte 6,8. Der Deckel 2 zeigt eine flache, oben glatte und durchgehende Oberseite und an der Vorderseite des Deckels 2 ragt eine Griffleiste 10 über etwa zwei Drittel bis drei Viertel der Höhe der Auskragung am Unterteil 1 über dieselbe hinab. Der untere Rand 11 dieser Griffleiste 10 kann deshalb von vorne mit dem Fingernagel vorzugsweise des Daumens oder aber des Zeige- oder Mittelfingers hintergriffen werden und dann kann der Deckel durch das Anheben dieser Griffleiste 10 aufgeschwenkt werden. Selbst mit Handschuhen kann diese Griffleiste 10 nach oben gezogen werden, weil das Material weichelastisch ist und somit der vordere Endbereich des Deckels 2 - vor der vorderen Auskragung am Unterteil 1 - leicht nach oben biegbar ist.

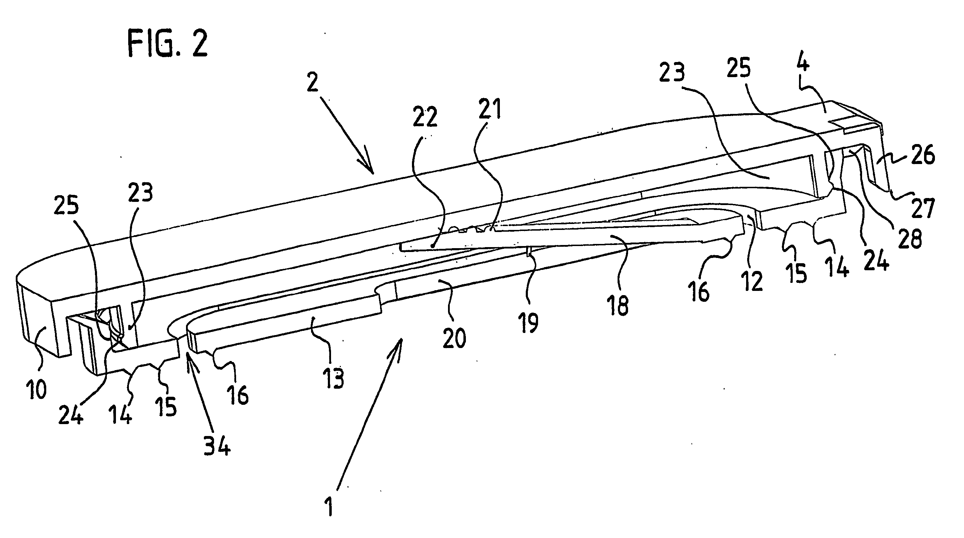

Die Figur 2 zeigt diesen Ausgiesser-Verschluss nach Figur 1 in geschlossenem Zustand, jedoch in einem Längsschnitt von schräg oben in perspektivischer Ansicht gesehen. Deshalb ist der Blick auf das Innere des Verschlusses freigegeben und man erkennt, dass die Grundplatte 9 des Unterteils 1 eine Durchflussöffnung 34 aufweist, deren Kontur oder deren Rand 12 im Grundriss die Form einer Null zeigt, das heisst eines Rechteckes mit abgerundeten Ecken. Innerhalb dieser lichten Durchflussöffnung 34 ist eine mit ihrem Aussenrand dem Innenrand 12 der Durchflussöffnung nachgeführte Wegreissplatte 13 über wenigstens zwei als Sollbruchstellen ausgelegte, hier jedoch nicht sichtbare Materialbrücken angeformt und gehalten. Die Unterseite dieser Wegreissplatte 13 verläuft bündig mit der Unterseite der Grundplatte 9. Sowohl die Unterseite der Grundplatte 9 wie auch die Unterseite der Wegreissplatte 13 ist mit Schweissrillen 14,15,16 versehen. Diese Schweissrillen verlaufen rundum die entsprechenden Teile entlang ihrer Ränder. Sie dienen dazu, bei der Verschweissung des Verschlusses mit einer Verbundpackung als Schweissmaterial, das heisst als aufzuschmelzendes Material zu dienen. Bei der standardgemäss mittels Ultraschall durchführten Schweissung werden sie vom Ultraschall erhitzt, schmelzen und sorgen für eine innige Verschmelzung des Verschlusses, das heisst des Unterteils 1 und der Wegreissplatte 13 mit der unter ihnen verlaufenden, durch den Ultraschall ebenfalls an die Schmelzgrenze gebrachten Kunststoff-Schicht auf dem Verbundlaminat. Innerhalb der Wegreissplatte 13 weist diese selbst abermals eine lichte Öffnung oder Aussparung 17 auf, innerhalb welcher eine Lasche 18 über einen schmalen Abschnitt mit dem hinteren Randabschnitt der lichten Öffnung 17 verbunden ist, und zwar in der Lage wie hier gezeigt, in welcher die Lasche 18 leicht schräg nach oben aus der lichten Öffnung 17 herausragt. Damit sie in dieser Lage gesichert ist, ist die Lasche 18 gegenüber den seitlichen Rändern 20 der lichten Öffnung 17 beidseits mittels feiner Stützrippen 19 abgestützt. Diese Stützrippen 19 können dabei mit den Rändern 20 der lichten Öffnung 17 verbunden sein, oder aber auch bloss lose auf ihnen aufliegen, je nach Ausführung. Sie haben bloss die Aufgabe, dafür zu sorgen, dass die Lasche 18 nicht in die lichte Öffnung hineinschwenkt, weil sie in dieser Lage nicht mehr ergriffen werden könnte. Eben zu diesem Ergreifen der schräg aus der lichten Öffnung 17 herausragenden Lasche 18 ist diese am Ende mit einer Riffelung 21 versehen, sodass dort ein eigentlicher Laschengriff 22 gebildet ist. Der nach unten ragende Kragen 23 an der Unterseite des Deckels 2 ist leicht vom Aussenrand des Deckels 2 nach innen versetzt und weist an seinem unteren, äusseren Kragenende einen Wulst 24 auf, welcher zum rundum erfolgenden Einklicken des Deckels 2 an der Innenseite der nach oben ragenden Auskragung 6,7,8 am Unterteil 1 dient. Diese Auskragung 6,7,8 ist demzufolge mit einem ebensolchen, in Schliesslage des Deckels 2 etwas höher als dessen Wulst 24 angeordneten Wulst 25 ausgerüstet, sodass der Wulst 24 am Deckelkragen 23 beim Zuschwenken des Deckels 2 über den Wulst 25 an der Innenseite der Auskragung 6,7,8 gleitet und hinter demselben einschnappt. Dieses Einklicken oder Einschnappen erfolgt unter leichter Deformation von Kragen 23 und Auskragung 7,6,8 gegeneinander. Jedenfalls wird der Deckel 2 in Schliesslage festgehalten und weist auch eine hinreichende Dichtigkeit auf. Das Öffnen durch Hochziehen der Griffleiste 10 gelingt deshalb, weil die Materialstärke sowohl des Deckels 2 wie auch des Unterteils 1 bloss in der Grössenordnung von etwa 1 mm liegt und die Teile deshalb entsprechend biegsam sind, sodass deren Geometrien hinreichend variabel sind, was das Ausklicken der Wulste 24,25 ermöglicht. Zu erwähnen ist noch, dass zuhinterst am Deckel 2 und zwischen den beiden Scharnierhebeln 4 ein nach unten ragender Zahn 26 angeformt ist. Wird der Deckel 2 um die wirksame Scharnierachse 5 geschwenkt, so streicht die spitze Endkante 27 dieses Zahns 26 in der hier gezeigten Darstellung im Uhrzeigersinn über den auskragenden Fortsatz 28 an der rückwärtigen Aussensseite der dort über ein Stück weit gerade verlaufenden Auskragung am Unterteil 1. Dieses Überstreichen des Fortsatzes 28 erfolgt unter elastischer Verformung des Zahns 26, was bewirkt, dass der Deckel 2 nicht mehr von selbst auf den Unterteil 1 zurückschwenken kann, weil dann der Zahn 26 am Fortsatz 28 ansteht und den Deckel 2 in mindestens um ca. 60° geöffneter Stellung blockiert.

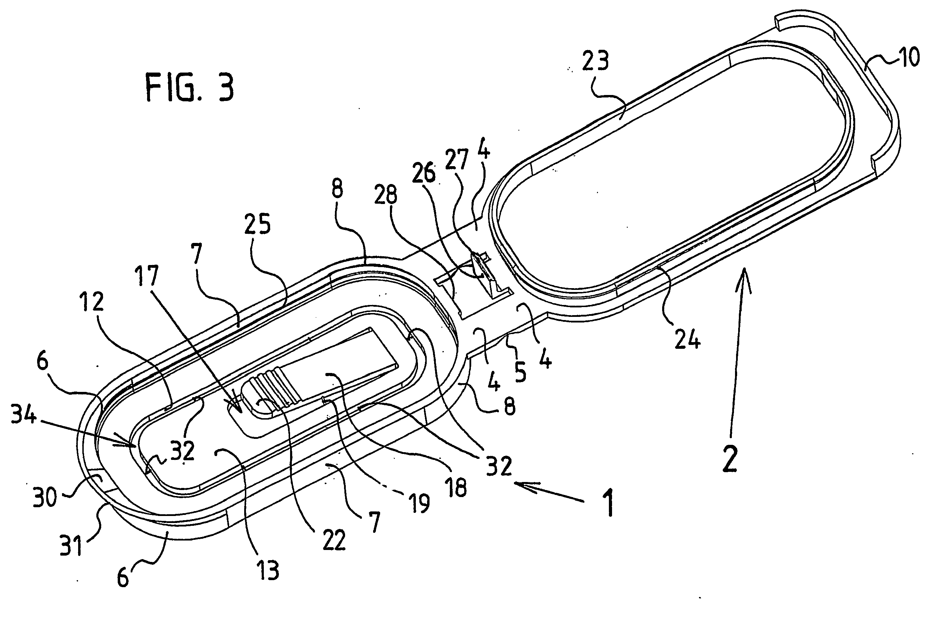

Die Figur 3 zeigt diesen Ausgiesser-Verschluss nach Figur 1 mit um 180° aufgeschwenktem Deckel 2, jedoch in noch ungeöffnetem Zustand der Verbundpackung, auf welche er aufgeschweisst oder aufgeklebt ist. In dieser Ansicht sieht man besonders gut einsehbar den eben beschriebenen Rückhaltezahn 26 mit seiner scharfen Endkante 27 am hinteren Ende des Deckels 2 sowie den mit ihm zusammenwirkenden, nach aussen ragenden Fortsatz 28 am oberen äusseren Rand der Auskragung hinten am Unterteil 1. Diese beiden Elemente sind in der sonst freibleibenden Aussparung 29 zwischen den Scharnierhebeln 4 an den beiden Teilen 1,2 angeordnet. Am Deckel 2 erkennt man den an seiner Unterseite angeformten, ca. 1 mm starken Kragen 23 mit seinem auf der Aussenseite entlanggeführten feinen Wulst 24. Als Gegenstück dazu wirkt der Wulst 25 oder eine leichte Vertiefung in der Aussenseite der Auskragung 6,7,8 am Unterteil 1. Diese Auskragung ist vorne am Unterteil etwas verlängert und gegen vorne geneigt, sodass damit eine Ausgiesslippe 30 mit scharfer Abrisskante 31 gebildet ist. Innerhalb des Unterteils 1 erkennt man dessen Grundplatte 9, aus welcher eine lichte Durchflussöffnung 34 ausgenommen ist, die vom Rand 12 begrenzt ist. Und innerhalb dieses Randes 12 ist die Wegreissplatte 13 zu sehen, die der Kontur dieses Randes 12 nachgeführt ist und im hier gezeigten Beispiel über insgesamt sechs feine Materialbrücken 32 mit demselben verbunden ist. Die Unterseite dieser Wegreissplatte 13 liegt bündig mit der Unterseite der Grundplatte 9 des Unterteils 1. Innerhalb der Wegreissplatte 13 weist diese ihrerseits eine Aussparung 17 auf, in welcher eine Grifflasche 18 angeformt ist, wobei diese nur an ihrer hinteren Seite am dortigen Rand der Aussparung 17 angeformt ist und deshalb und aufgrund der Elastizität des Materials, welches ja bloss eine Stärke von ca. 1mm aufweist, ohne weiteres nach oben schwenkbar ist. Damit diese Lasche 18 nicht ganz in die Aussparung 17 in der Wegreissplatte 13 abschwenken kann, ist sie an ihren beiden Längsrändem mit Stützrippen 19 ausgerüstet, mittels derer sie am Rand der Aussparung 17 abgestützt ist, sodass ein Abschwenken in die Aussparung 17 verhindert ist. Diese Stützrippen 19 können an der Aussparung 17 angeformt sein, jedoch auch bloss lose auf ihr abgestützt sein. Am vorderen Ende der Grifflasche 18 ist diese mit einer Riffelung 21 versehen. Sie kann an diesem vorderen Ende deshalb leicht und rutschsicher ergriffen und hernach hochgeschwenkt werden. Zum effektiven Öffnen des Verschlusses, das heisst zum Öffnen einer mit diesem Verschluss ausgerüsteten Verbundpackung wird sodann die Lasche 18 an ihrem Ende 22 ergriffen und hochgeschwenkt. Mit einer herzhaften Reissbewegung wird sodann die ganze Wegreissplatte 13 unter Bruch der Materialbrücken 32 aus dem Unterteil 1 des Verschlusses losgerissen. Entsprechend wird das Stück Dichtfolie, auf welches die Unterseite der Wegreissplatte geschweisst oder geleimt wurde, aus dem Verbund herausgerissen und gibt das herausgestanzte Loch frei, über welches die Dichtfolie geklebt oder geschweisst wurde.

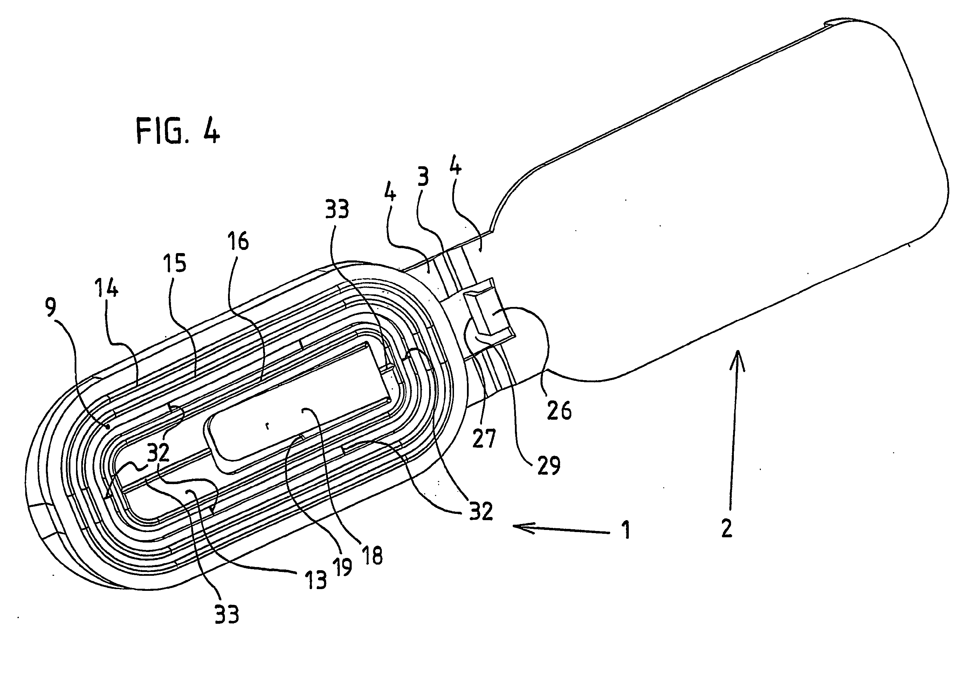

Die Figur 4 zeigt den Ausgiesser-Verschluss noch mit um 180° aufgeschwenktem Deckel 2 und in noch ungeöffnetem Zustand der Verbundpackung, das heisst mit intakter Wegreissplatte 13, jetzt aber in perspektivischer Ansicht von unten gesehen. Hier erkennt man wiederum, dass die Oberseite des Deckels 2 durchwegs glatt ausgeführt ist, um der Forderung Rechnung zu tragen, einen möglichst flachen Verschluss von minimaler Bauhöhe zu schaffen. Zwischen den Scharnierhebeln 4 erkennt man die Aussparung 29 mit dem in sie ragenden Zahn 26, sowie das durch eine Dünnstelle realisierte Filmscharnier 3. Auf der hier einsehbaren Unterseite des Verschluss-Unterteils 1 erkennt man die beiden Schweissrillen 14,15 an der Grundplatte 9, sowie auch die Schweissrille 16 an der Wegreissplatte 13. Wie man sieht, erstreckt sich eine weitere Schweissrille 33 in Längsrichtung mittig über die Wegreissplatte 13. Es ist wichtig, dass die Wegreissplatte 13 zugkraftschlüssig mit der Dichtfolie verschweisst oder verklebt wird, welche über das ausgestanzte Loch geklebt wird, damit bei ihrem Wegreissen dieses Dichtfolienstück auch tatsächlich herausgerissen wird. Weiter sieht man in dieser Figur auch die Grifflasche 18 mit ihren Stützrippen 19, sowie die Materialbrücken 32, über welche die Wegreissplatte 13 an der Grundplatte 9 des Unterteils 1 gehalten ist.

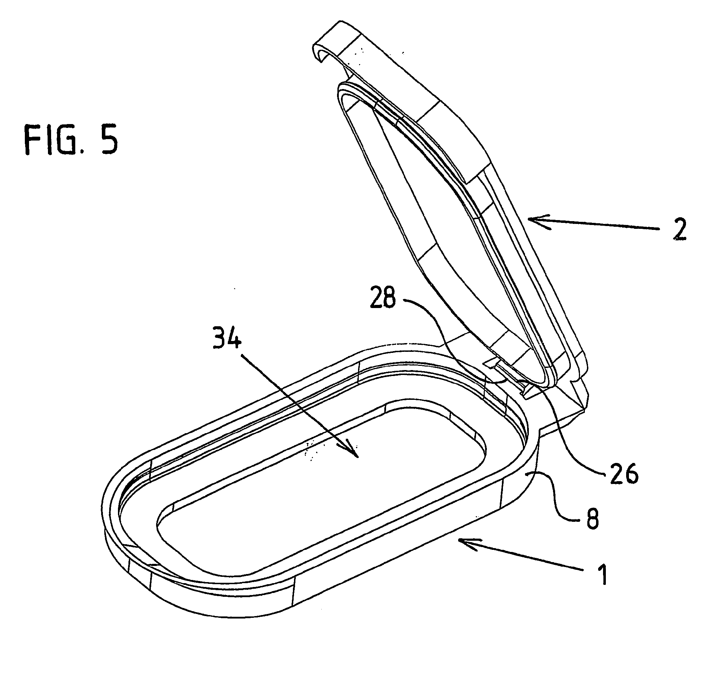

Die Figur 5 zeigt den Ausgiesser-Verschluss nach Figur 1 schliesslich noch mit um etwa 60° aufgeschwenktem Deckel 2 und in geöffnetem Zustand der Verbundpackung, das heisst mit weggerissener Wegreissplatte. Die lichte Durchflussöffnung 34 ist dadurch vollständig freigegeben und ein kongruentes Stück Dichtfolie ist aus der Dichtfolie über dem Verbundmaterial herausgerissen worden, sodass die lichte Durchflussöffnung 34 voll genutzt werden kann. Wie man hier sieht, steht der Zahn 26 am Verschlussdeckel 2 am Fortsatz 28 an der Hinterseite der Auskragung 8 an und hält den Deckel 2 in der gezeigten Schwenklage offen.

### Ziffernverzeichnis

- 1: Unterteil
- 2: Deckel
- 3: Filmschamiere zwischen Deckel und Unterteil
- 4: Scharnierhebel
- 5: Scharnierachse
- 6: gekrümmter Abschnitt Auskragung an Unterteil vorne
- 7: gerader Abschnitt Auskragung an Unterteil seitlich
- 8: gekrümmter Abschnitt Auskragung an Unterteil hinten
- 9: Grundplatte Unterteil
- 10: Griffleiste an Deckel
- 11: Unterer Rand der Griffleiste
- 12: Innerer Rand der lichten Durchflussöffnung
- 13: Wegreissplatte
- 14: äussere Schweissrille an Grundplatte
- 15: innere Schweissrille an Grundplatte
- 16: Schweissrille an Wegreissplatte
- 17: Aussparung in Wegreissplatte
- 18: Lasche
- 19: Stützrippen an Lasche
- 20: Innenrand der Aussparung in Wegreissplatte
- 21: Riffelung an Laschengriff
- 22: Laschengriff
- 23: Kragen an Deckelunterseite
- 24: Wulst aussen an Kragen
- 25: Wulst an innenseite Auskragung an Unterteil
- 26: Zahn an Deckel
- 27: scharfe Kante an Zahn
- 28: Fortsatz gegenüber Zahn
- 29: Aussparung zwischen den Scharnierhebeln
- 30: Ausgiesslippe
- 31: Scharfe Abreisskante der Ausgiesslippe
- 32: Materialbrücken der Wegreissplatte
- 33: Schweissrille längs auf Wegreissplatte
- 34: lichte Durchflussöffnung

## Patentansprüche

1. Flacher und niedriger Ausgiesser-Verschluss für Verbundpackungen oder mit einer Dichtfolie verschlossene Behälteröffnungen, bestehend aus einem Unterteil (1), welcher mit der Unterseite seiner Grundplatte (9) auf eine Verbundpackung oder Dichtfolie aufzuschweissen oder aufzuleimen bestimmt ist, wobei die Grundplatte (9) eine Durchflussöffnung (34) aufweist sowie eine diese Grundplatte (9) aussen umlaufende, nach oben ragende Auskragung (6,7,8), sowie aus einem zu diesem Unterteil (1) über ein Scharnier (5) schwenkbar angeformten Deckel (2) zum Aufschwenken und dichtenden Niederschwenken auf den Unterteil (1), mit in die Auskragung (6,7,8) am Unterteil (1) einpassendem rundumlaufenden Kragen (23) auf der Unterseite des Deckels (2), wobei an der Grundplatte (9) in deren lichter Durchflussöffnung (34) eine mit ihrem Aussenrand dem Innenrand (12) der Durchflussöffnung (34) nachgeführte Wegreissplatte (13) über Materialbrücken (32) als Sollbruchstellen angeformt ist, sodass die Unterseite der Wegreissplatte (13) mit der Unterseite der Grundplatte (9) bündig verläuft, ***dadurch gekennzeichnet,* dass** die Wegreissplatte (13) eine Aussparung (17) aufweist, in welcher an einer Randstelle eine Lasche (18) angeformt ist, an welcher die Wegreissplatte (13) nach Aufschwenken des Deckels (2) unter Bruch der Materialbrücken (32) aus der Durchflussöffnung (34) herausreissbar ist.

2. Flacher und niedriger Ausgiesser-Verschluss für Verbundpackungen oder mit einer Dichtfolie verschlossene Behälteröffnungen nach Anspruch 1, ***dadurch gekennzeichnet,* dass** die Lasche an ihren Längsseiten mit Stützrippen (19) versehen ist, sodass sie in schräg nach oben ragender Position in der Durchflussöffnung (34) gehalten ist.

3. Flacher und niedriger Ausgiesser-Verschluss für Verbundpackungen oder mit einer Dichtfolie verschlossene Behälteröffnungen nach einem der vorangehenden Ansprüche, ***dadurch gekennzeichnet,* dass** längs der Kragenaussenseite des Kragens (23) an der Deckelunterseite ein Wulst (24) angeformt ist, welcher bei niedergeschwenktem Deckel (2) hinter einen Wulst (25) eingeklickt zu liegen kommt, welcher längs der Innenseite der Auskragung (6,7,8) am Unterteil (1) des Verschlusses angeformt ist.

4. Flacher und niedriger Ausgiesser-Verschluss für Verbundpackungen oder mit einer Dichtfolie verschlossene Behälteröffnungen nach einem der vorangehenden Ansprüche, ***dadurch gekennzeichnet,* dass** das Scharnier (3) an Deckel (2) und Unterteil (1) je zwei Scharnierhebel (4) aufweist, die zwischen sich einen Freiraum (29) offenlassen, und dass in diesen Freiraum (29) ein am hinteren Deckelrand angeformter, abgewinkelter Zahn (26) mit scharfem Rand (27) ragt, welcher beim Zuschwenken des Deckels (2) an einem auf der anderen Seite in den Freiraum (29) ragenden Fortsatz (28) am oberen äusseren Rand der Auskragung (8) am Unterteil (1) anschlägt, wobei der Fortsatz (28) vom Rand (27) des Zahns (26) unter leichter elastischer Deformation des Zahns (26) passierbar beziehungsweise überstreichbar ist.

5. Flacher und niedriger Ausgiesser-Verschluss für Verbundpackungen oder mit einer Dichtfolie verschlossene Behälteröffnungen nach einem der vorangehenden Ansprüche, ***dadurch gekennzeichnet,* dass** der Deckel (2) einzig aus einer durchgehenden Deckelplatte besteht, die oben flach und glatt ist und an ihrer Unterseite einen gegenüber dem Deckelrand nach innen zurückversetzten Kragen (23) mit seitlich aus der Aussenseite längs des Kragens ver laufendem Wulst (24) aufweist, und dass am vorderen Ende des Deckels (2) eine nach unten ragende Griffleiste (10) angeformt ist, die zwei Drittel bis drei Viertel so hoch wie die Höhe des Auskragung (6,7,8) am Unterteil (1) ist.

6. Flacher und niedriger Ausgiesser-Verschluss für Verbundpackungen oder mit einer Dichtfolie verschlossene Behälteröffnungen nach einem der vorangehenden Ansprüche, ***dadurch gekennzeichnet,* dass** die Unterseite des Unterteils (1) und die Unterseite der Wegreissplatte (13) mit Schweissrillen (14,15;16,32) versehen sind, als mit der Dichtfolie auf der Verbundpackung zu verschweissendes Material,

7. Flacher und niedriger Ausgiesser-Verschluss für Verbundpackungen oder mit einer Dichtfolie verschlossene Behälteröffnungen nach einem der vorangehenden Ansprüche, ***dadurch gekennzeichnet,* dass** die Auskragung (6,7,8) auf der Grundplatte (9) des Unterteils (1) am vorderen Ende des Unterteils (1) in eine Ausgiess-Lippe (30) ausgeformt ist, welche einen scharfe Abrisskante (31) aufweist.

8. Flacher und niedriger Ausgiesser-Verschluss für Verbundpackungen oder mit einer Dichtfolie verschlossene Behälteröffnungen nach einem der vorangehenden Ansprüche, ***dadurch gekennzeichnet,* dass** die gesamte Höhe des Verschlusses weniger als ein Zehntel seiner gesamten Länge und weniger als ein Drittel seiner Breite ausmacht.

## Claims

1. Flat and low pourer-closure for composite packs or container openings, which are closed with a thick foil, comprising a bottom part (1), which, with the bottom side of its base plate (9), is intended to be welded or glued on a composite pack or thick foil, where the base plate (9) has a drain opening (34) and also an upwardly jutting projection (6,7,8), which surrounds this base plate (9) on the outside, and a molded cover (2), which can be swiveled through a hinge (5) towards the bottom part (1) for swiveling up and tightly swiveling down on the bottom part (1) with collar (23), running all around on the bottom side of the cover (2), which corresponds with the projection (6,7,8) on the bottom part (1), wherein on the base plate (9), in whose internal drain opening (34) a break off plate (13) is formed with its outer edge connected to the inner edge (12) of the drain opening (34) through material bridges (32) as breaking points, so that the bottom side of the break off plate (13) runs flush with the bottom side of the base plate (9), **characterized in that** the break off plate (13) has a recess (17), in which, at an edge position, a tongue (18) is formed, at which the break off plate (13) can be torn off by breaking the material bridges (32) from the drain opening (34) after swivelling up of the cover (2).

2. Flat and low pourer-closure for composite packs or container openings, which are closed with a thick foil, according to Claim 1, **characterized in that** the tongue is provided with supporting ribs (19) at its length side so that it is held in the slanted up position in the drain opening (34).

3. Flat and low pourer-closure for composite packs or container openings, which are closed with a thick foil, according to any of the previous Claims, **characterized in that** a bead (24) is formed along the length of the outer side of the collar (23) on the bottom side of the cover, said bead being ready for clicking- in behind a bead (25) when the cover is swiveled down, bead (25) being formed along the length of the inner side of the projection (6,7,8) on the bottom part (1) of the closure.

4. Flat and low pourer-closure for composite packs or container openings, which are closed with a thick foil, according to any of the previous Claims, **characterized in that** the hinge (3) on cover (2) and bottom part (1) has two hinge levers (4) respectively, which between themselves open a free space (29), and that in this free space (29), a tooth (26) with sharp edge (27), which is formed at a back edge of the cover, projects and, while the cover (2) swivels shut, the tooth strikes against an extension (28), which projects in the free space (29) on the other side and is at the top outer edge of the projection (8)on the bottom part (1), wherein, under light elastic deformation of the tooth (26), the edge (27) of the tooth (26) can pass over or top the extension (28).

5. Flat and low pourer-closure for composite packs or container openings, which are closed with a thick foil, according to any of the previous Claims, **characterized in that** the cover (2) only is made up of a continuous cover plate, which is smooth and flat at top and has at its bottom side a collar (23), which is moved back inside opposite the cover edge and has a bead (24), which runs along the length of the collar side-wise from the outer side, and that at the front end of the cover (2), a holding strip (10), projecting downwards, is formed, which is two thirds to three quarter as high as the height of the projection (6,7,8) on the bottom part (1).

6. Flat and low pourer-closure for composite packs or container openings, which are closed with a thick foil, according to any of the previous Claims, **characterized in that** the bottom side of the bottom part (1) and the bottom side of the break off plate (13) are provided with welding ridges (14, 15; 16, 32) as material for welding with the thick foil on that composite pack.

7. Flat and low pourer-closure for composite packs or container openings, which are closed with a thick foil, according to any of the previous Claims, **characterized in that** the projections (6, 7, 8) on the base plate (9) of the bottom part (1) is formed in to a pourer lip (30) at the front end of the bottom part (1), said pourer lip having a sharp break off edge (31).

8. Flat and low pourer-closure for composite packs or container openings, which are closed with a thick foil, according to any of the previous Claims, **characterized in that** the total height of the closure constitutes less than one tenth of its total length and less than one third of its width.

## Revendications

1. Dispositif de fermeture plat et bas à verseur pour des emballages composites ou pour des ouvertures de récipients fermées par une feuille d'étanchéité, composé d'une partie inférieure (1), destinée à être soudée ou collée avec la face inférieure de sa plaque de base (9) sur un emballage composite ou une feuille d'étanchéité, la plaque de base (9) comportant une ouverture d'écoulement (34) ainsi qu'une saillie (6, 7, 8) entourant extérieurement cette plaque de base (9) et dépassant vers le haut ainsi que d'un couvercle (2) pivotant rattaché par une charnière (5) à cette partie inférieure (1) pour permettre l'ouverture par pivotement et l'abaissement étanche par pivotement sur la partie inférieure (1), avec un rebord périphérique (23) disposé sur la surface inférieure du couvercle (2) et s'insérant dans la saillie (6, 7, 8), sur la partie inférieure (1), cependant que, sur la plaque de base (9), dans son ouverture d'écoulement (34), une plaque d'arrachement (13), épousant avec son bord extérieur le bord intérieur (12) de l'ouverture d'écoulement (34), est raccordée par des pontets (32) de matière formant des points de rupture imposée, de sorte que la surface inférieure de la plaque d'arrachement (13) s'étend jointivement avec la surface inférieure de la plaque de base (9), **caractérisé en ce que** la plaque d'arrachement (13) présente un évidement (17), dans lequel, à un endroit latéral, une patte (18) est rattachée, par laquelle, une fois que le couvercle (2) s'est ouvert par pivotement en rompant les pontets de matière (32), la plaque d'arrachement (13) peut être arrachée de l'ouverture d'écoulement (34).

2. Dispositif de fermeture plat et bas à verseur pour des emballages composites ou pour des ouvertures de récipients fermées par une feuille d'étanchéité suivant la revendication 1, **caractérisé en ce que** la patte (18) est munie, sur ses côtés longs, de tiges d'appui (19), de sorte qu'elle est maintenue, en position dépassant obliquement vers le haut, dans l'ouverture de passage (34).

3. Dispositif de fermeture plat et bas à verseur pour des emballages composites ou pour des ouvertures de récipients fermées par une feuille d'étanchéité suivant une des revendications précédentes, **caractérisé en ce que**, le long de la surface extérieure du rebord (23), il est rattaché sur la surface inférieure du couvercle un bourrelet (24), qui, quand le couvercle (2) a pivoté vers le bas, vient s'encranter derrière un bourrelet (25), qui est rattaché le long de la surface intérieure de la saillie (6, 7, 8) sur la partie inférieure (1) du dispositif de fermeture.

4. Dispositif de fermeture plat et bas à verseur pour des emballages composites ou pour des ouvertures de récipients fermées par une feuille d'étanchéité suivant une des revendications précédentes, **caractérisé en ce que** la charnière (3) disposée sur le couvercle (2) et la partie inférieure (1) comporte chaque fois deux leviers (4) de charnière, qui laissent entre eux un espace libre (29) et que, dans cet espace libre, une dent coudée(26), rattachée au bord arrière du couvercle, dépasse avec un bord vif (27), qui, au moment de la fermeture du couvercle (2) par pivotement, vient buter contre un prolongement (28) dépassant de l'autre côté, dans l'espace libre (29) et disposé sur le bord supérieur extérieur de la saillie (8) de la partie inférieure (1), cependant que le prolongement (28) peut être passé et/ou surmonté par le bord (27) de la dent (26), avec une légère déformation élastique de la dent (26).

5. Dispositif de fermeture plat et bas à verseur pour des emballages composites ou pour des ouvertures de récipients fermées par une feuille d'étanchéité suivant une des revendications précédentes, **caractérisé en ce que** le couvercle (2) est formé uniquement d'une plaque de couvercle s'étendant de bout en bout, qui est dans le haut plate et lisse et comporte sur sa surface inférieure un rebord (23) en retrait vers l'intérieur par rapport au bord du couvercle, avec un bourrelet (24), s'étendant latéralement à partir de la surface extérieure le long du rebord, et qu'à l'extrémité avant du couvercle (2) est rattachée une baguette de prise (10) dépassant vers le bas, dont la hauteur est de deux tiers à trois quarts de celle de la saille (6, 7, 8), disposée sur la partie inférieure (1).

6. Dispositif de fermeture plat et bas à verseur pour des emballages composites ou pour des ouvertures de récipients fermées par une feuille d'étanchéité suivant une des revendications précédentes, **caractérisé en ce que** la surface inférieure de la partie inférieure (1) et la surface inférieure de la plaque d'arrachement (13) sont munies de rainures de soudage (14, 15; 16, 32), constituant un matériel à souder avec la feuille d'étanchéité sur l'emballage composite.

7. Dispositif de fermeture plat et bas à verseur pour des emballages composites ou pour des ouvertures de récipients fermées par une feuille d'étanchéité suivant une des revendications précédentes, **caractérisé en ce que** la saillie (6, 7, 8) disposée sur la plaque de base (9) de la partie inférieure (1) forme à l'extrémité avant de la partie inférieure (1) une lèvre de versage (30), qui présente une arête vive de déchirement (31).

8. Dispositif de fermeture plat et bas à verseur pour des emballages composites ou pour des ouvertures de récipients fermées par une feuille d'étanchéité suivant une des revendications précédentes, **caractérisé en ce que** la hauteur totale du dispositif de fermeture représente moins d'un dixième de sa longueur totale et moins d'un tiers de sa largeur.
